# EUROPEAN PATENT APPLICATION

(11) **EP 3 613 730 A1**
(43) Date of publication of application: **26.02.2020**
(21) Application number: 18788214.7
(22) Date of filing: 18.04.2018
(51) Int. Cl.: C07D 213/71, C07D 401/04

(54) **METHOD FOR PRODUCING PYRIDINE COMPOUND**

(30) Priority: 19.04.2017 JP 2017082678
(71) Applicant: Sumitomo Chemical Company, Limited, Tokyo 104-8260 (JP)
(72) Inventor: KIMURA, Takahiro, Takarazuka-shi Hyogo 665-8555 (JP); KOJIMA, Yasuhiro, Takarazuka-shi Hyogo 665-8555 (JP); HAYASHI, Yumi, Takarazuka-shi Hyogo 665-8555 (JP); MAEHATA, Ryota, Takarazuka-shi Hyogo 665-8555 (JP)
(74) Representative: Vossius & Partner Patentanwälte Rechtsanwälte mbB
(86) International application number: PCT/JP2018/015929
(87) International publication number: WO 2018/194077

(57) **Abstract**

The present invention provides a method for producing a compound represented by formula (4), which is useful as, for example, an agrochemical or an intermediate for agrochemical production, the method including the step of reacting a compound represented by formula (2) with an organic oxidant. [In the formulae, Q represents a pyridyl group, etc.; R¹ represents a C₁₋₆ alkyl group optionally having one or more halogen atoms; R² represents, for example, a C₁₋₆ alkyl group optionally having one or more halogen atoms; R³, R⁴, and R⁵ each represent a hydrogen atom, etc.; and n is 0, etc.)

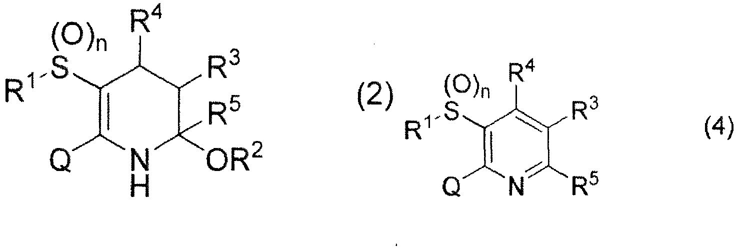

## Description

### TECHNICAL FIELD

This application claims priority to and the benefit of Japanese Patent Application No. 2017-082678 filed on April 19, 2017, the entire contents of which are incorporated herein by reference.

The present invention relates to a method for preparing a pyridine compound.

### BACKGROUND ART

A pyridine compound is an important compound group as a final product and its preparation intermediate for pharmaceuticals, pesticides, and liquid crystals, etc. For example, Patent Document 1 discloses a compound having a pyridine structure which is useful as an agent for controlling a harmful arthropod and its preparation method, in which the method comprises adding an enamine compound to acrolein, and then reacting the obtained compound with methanesulfonyl chloride and triethylamine. However, the method of Patent Document 1 provides the pyridine compound at only a yield of less than 30%, and thus a more efficient process for making a pyridine ring has been required.

For a process for preparing a pyridine compound, a process of reacting a 3-aminocrotonic acid ethyl ester with acrolein to obtain a dihydropyridine compound and oxidizing the dihydropyridine compound with chloranil is also known (see, for example, Patent Document 2). However, a dihydropyridine compound, which is needed to isolate in the process disclosed in Patent Document 2, is commonly sensitive to light or heat and has a character of being degradated by a nucleophile such as water and an alcohol. Hence, a pyridine compound originating from the dihydropyridine compound is produced at only a yield of less than 30%.

Further, a process of reacting an α-hydroxydihydropyran compound with ammonium acetate to obtain an α-hydroxytetrahydropyridine compound, dehydrating said compound under the presence of 4-toluenesulfonic acid to obtain a dihydropyridine compound, and then oxidizing the dihydropyridine compound with 2,3-dichloro-5,6-dicyano-1,4-benzoquinone is also known (see, for example, Non-Patent Document 1). However, Non-Patent Document 1 indicates that a yield of the reaction for preparing the α-hydroxytetrahydropyridine compound greatly varies depending upon the type of a substance. In particular, when an α-hydroxydihydropyran compound has an electron-withdrawing group at its 6-position, an α-hydroxytetrahydropyridine compound that is an intermediate for constructing a pyridine ring can be obtained at only a low yield because a byproduct is generated due to a very slow reaction for producing the α-hydroxytetrahydropyridine compound.

### CITATION LIST

### PATENT DOCUMENT

Patent Document 1: WO 2016/121969 pamphlet
Patent Document 1: JP-H5-310696 A1

### NON-PATENT DOCUMENT

Non-Patent Document 1: Valentine G. Nenajdenko etc., Efficient route to 6-CF3-substituted nicotinic acid derivatives, Journal of Fluorine Chemistry, Vol. 127, p. 865-873, 2006

### SUMMARY OF THE INVENTION

### PROBLEMS TO BE SOLVED BY INVENTION

An object of the present invention is to provide a method for efficiently preparing a pyridine compound without a need for making a dihydropyridine compound or an α-hydroxytetrahydropyridine which is known to be commonly sensitive to light or heat and unstable to a nucleophile such as water.

### MEANS TO SOLVE PROBLEMS

The present inventors have intensively studied to find out a method for preparing a pyridine compound. As a result, they have found out that a pyridine compound can be efficiently prepared by reacting a ketone compound with an α,β-unsaturated aldehyde compound and ammonia or an ammonium salt to obtain a stable cyclic hemiaminal ether compound and then reacting the obtained compound with an organic oxidizing agent.

Namely, the present invention is as follows.
[1] A method for preparing a compound represented by formula (4) : 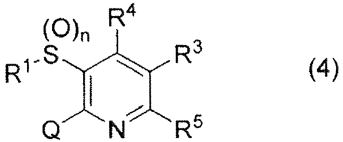 wherein,
   Q represents a pyridyl group, a pyrimidinyl group, a pyrazinyl group, or a pyridazinyl group, wherein the pyridyl group, the pyrimidinyl group, the pyrazinyl group and the pyridazinyl group each independently may have optionally one or more substituents that are inactivated for an oxidation reaction;
   R¹ represents a C1-C6 alkyl group optionally having one or more halogen atoms;
   R³ represents a C1-C6 alkyl group, an aryl group, a heteroaryl group, wherein the C1-C6 alkyl group, the aryl group and the heteroaryl group each independently may have optionally one or more substituents that are inactivated for an oxidation reaction, OR¹⁰, NR¹⁰R¹¹, NR¹⁰OR¹¹, S(O)_{y}R¹⁰, NR¹⁰C(O)R¹¹, NR¹⁰C(O)OR¹¹, a halogen atom, or a hydrogen atom;
   R⁴ and R⁵ are identical to or different from each other and represent a C1-C6 alkyl group optionally having one or more halogen atoms, an aryl group, a heteroaryl group, wherein the aryl group and the heteroaryl group each independently may have optionally one or more substituents that are inactivated for an oxidation reaction, or a hydrogen atom;
   R¹⁰ and R¹¹ are identical to or different from each other and represent a C1-C6 alkyl group optionally having one or more substituents that are inactivated for an oxidation reaction; and
   n and y each independently represent 0, 1, or 2, which comprises a step of reacting a compound represented by formula (2): 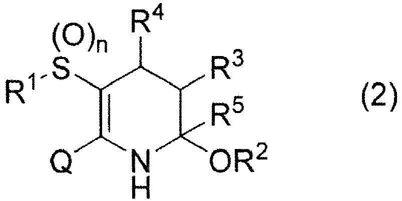
   wherein, R² represents a C1-C6 alkyl group optionally having one or more halogen atoms, and the other symbols have the same meanings as defined above,
   with an organic oxidizing agent selected from the group consisting of a quinone oxidizing agent, an N-halosuccinimide oxidizing agent, a hypervalent iodine oxidizing agent, and an N-oxide compound.
[2] The method according to [1], wherein R³ represents OR¹⁰, NR¹⁰R¹⁰, NR¹⁰OR¹¹, S(O)_{y}R¹⁰, or a hydrogen atom, and R⁴ and R⁵ represent a hydrogen atom.
[3] The method according to [1], wherein R¹ represents an ethyl group, R² represents a C1-C6 alkyl group, R³ represents a hydrogen atom or a benzyloxy group, R⁴ and R⁵ represent a hydrogen atom, n represents 2, and Q represents a pyridyl group, a pyrimidinyl group, a pyrazinyl group, or a pyridazinyl group, wherein the pyridyl group, the pyrimidinyl group, the pyrazinyl group and the pyridazinyl group each independently may have optionally a halogen atom or a C1-C6 alkoxy group optionally substituted with a halogen atom.
[4] The method according to any one of [1] to [3], wherein the organic oxidizing agent is a p-quinone oxidizing agent represented by formula (7): 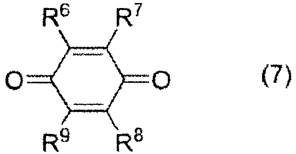 wherein, R⁶, R⁷, R⁸, and R⁹ each independently represent a hydrogen atom, a methyl group, a t-butyl group, a halogen atom, or cyano group,
   an o-quinone oxidizing agent represented by formula (8): 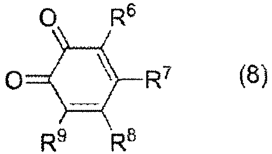 wherein the symbols have the same meanings as defined above, or an N-halosuccinimide oxidizing agent.
[5] The method according to any one of [1] to [4], which comprises a step of reacting a compound represented by formula (6): 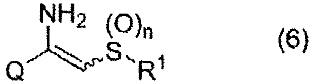 wherein the symbols have the same meanings as defined above, with a compound represented by formula (3): 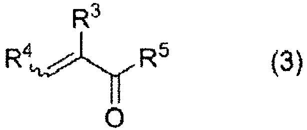 wherein the symbols have the same meanings as defined above, under the presence of an alcohol represented by
   R²OH
   wherein the symbols have the same meanings as defined above, to obtain the compound represented by formula (2).
[6] The method according to any one of [1] to [4], which comprises a step of reacting a compound represented by formula (5a): 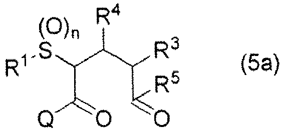 wherein the symbols have the same meanings as defined above, or a compound represented by formula (5b): 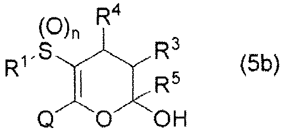 wherein the symbols have the same meanings as defined above, with ammonia or an ammonium salt under the presence of an alcohol represented by
   R²OH
   wherein the symbol has the same meaning as defined above, to obtain the compound represented by formula (2).
[7] The method according to [5], which comprises a step of reacting a compound represented by formula (1): 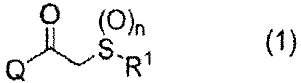 wherein the symbols have the same meaning as defined above, with ammonia or an ammonium salt to obtain the compound represented by formula (6).
[8] The method according to [6], which comprises a step of reacting a compound represented by formula (1): 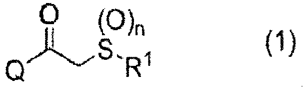 wherein the symbols have the same meanings as defined above, with a compound represented by formula (3): 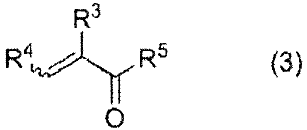 wherein the symbols have the same meaning as defined above, to obtain the compound represented by formula (5a) or the compound represented by formula (5b).

### MODE FOR CARRYING OUT THE INVENTION

The groups as used herein are explained as follows.

The "optionally having substituent(s)" as used herein represents that a particular compound has some substituent(s) or no substituent. When a compound has two or more substituents, the substituents may be identical to or different from each other.

The substituent that is inactivated for an oxidation reaction, which the pyridyl group, the pyrimidinyl group, the pyrazinyl group, or the pyridazinyl group each represented as "Q" may have optionally, includes, for example, a C1-C6 alkyl group optionally having one or more halogen atoms, a C1-C6 alkylsulfonyl group optionally having one or more halogen atoms, a C6-C10 arylsulfonyl group optionally having one or more C1-C6 alkyl groups, a C1-C6 alkoxy group optionally substituted with a halogen atom, a C6-C10 arylsulfonyl group optionally having one or more nitro groups, OR¹², NR¹³R¹⁴, a cyano group, a nitro group, and a halogen atom, preferably a C1-C6 alkyl group optionally having one or more halogen atoms, OR¹², a nitro group, and a halogen atom, more preferably OR¹², and a halogen atom.

R¹² represents a C1-C10 chain hydrocarbon group optionally having one or more halogen atoms, a (C1-C5 alkyloxy)C2-C5 alkyl group having one or more halogen atoms, a (C1-C5 alkylsulfanyl)C2-C5 alkyl group having one or more halogen atoms, a (C1-C5 alkylsulfinyl)C2-C5 alkyl group having one or more halogen atoms, a (C1-C5 alkylsulfonyl)C2-C5 alkyl group having one or more halogen atoms, a C1-C6 alkylsulfonyl group optionally having one or more halogen atoms, a C6-C10 arylsulfonyl group optionally having one or more C1-C6 alkyl groups, a C6-C10 arylsulfonyl group optionally having one or more nitro groups, a C1-C6 alkyl group optionally having one or more hetero atoms, a (C3-C7 cycloalkyl)C1-C3 alkyl group having one or more substituents selected from Group G, or a C3-C7 cycloalkyl group having one or more substituents selected from Group G.
Group G: the group consisting of a halogen atom, and a C1-C6 haloalkyl group.

R¹³ and R¹⁴ are identical to or different from each other, and represent a C1-C6 chain hydrocarbon group optionally having one or more halogen atoms.

The substituent that is inactivated for an oxidation reaction, which the C1-C6 alkyl group represented as "R³" may have optionally, includes, for example, a C1-C6 alkyloxy group optionally having one or more halogen atoms, a C3-C6 alkenyloxy group optionally having one or more halogen atoms, a C3-C6 alkynyloxy group optionally having one or more halogen atoms, a C1-C6 alkylsulfanyl group optionally having one or more halogen atoms, a C1-C6 alkylsulfinyl group optionally having one or more halogen atoms, a C1-C6 alkylsulfonyl group optionally having one or more halogen atoms, a C3-C6 cycloalkyl group optionally having one or more halogen atoms, a cyano group, and a halogen atom.

The substituent that is inactivated for an oxidation reaction, which the aryl group or the heteroaryl group each represented as "R³" may have optionally, includes, for example, a C1-C6 chain hydrocarbon group optionally having one or more halogen atoms, a C1-C6 alkyloxy group optionally having one or more halogen atoms, a C3-C6 alkenyloxy group optionally having one or more halogen atoms, a C3-C6 alkynyloxy group optionally having one or more halogen atoms, a C1-C6 alkylsulfanyl group optionally having one or more halogen atoms, a C1-C6 alkylsulfinyl group optionally having one or more halogen atoms, a C1-C6 alkylsulfonyl group optionally having one or more halogen atoms, NR¹⁵R¹⁶, C(O)R¹⁵, OC(O)R¹⁵, C(O)OR¹⁵, a cyano group, a nitro group, and a halogen atom.

R¹⁵ and R¹⁶ are identical to or different from each other, and represent a C1-C6 alkyl group optionally having one or more halogen atoms.

The substituent that is inactivated for an oxidation reaction, which the aryl group or the heteroaryl group each represented as "R⁴" and "R⁵" may have optionally, includes, for example, a C1-C6 chain hydrocarbon group optionally having one or more halogen atoms, a C1-C6 alkyloxy group optionally having one or more halogen atoms, a C3-C6 alkenyloxy group optionally having one or more halogen atoms, a C3-C6 alkynyloxy group optionally having one or more halogen atoms, a C1-C6 alkylsulfanyl group optionally having one or more halogen atoms, a C1-C6 alkylsulfinyl group optionally having one or more halogen atoms, a C1-C6 alkylsulfonyl group optionally having one or more halogen atoms, NR¹⁷R¹⁸, C(O)R¹⁷, OC(O)R¹⁷, C(O)OR¹⁷, a cyano group, a nitro group, and a halogen atom.

R¹⁷ and R¹⁸ are identical to or different from each other, and represent a C1-C6 alkyl group optionally having one or more halogen atoms.

The substituent that is inactivated for an oxidation reaction, which the C1-C6 alkyl group represented as "R¹⁰" and "R¹¹" may have optionally, includes, for example, an aryl group optionally having one or more substituents selected from Group D, a heteroaryl group optionally having one or more substituents selected from Group D, a halogen atom, and S(O)₂R¹⁹.
Group H: the group consisting of a C1-C6 chain hydrocarbon group optionally having one or more halogen atoms, a C1-C6 alkyloxy group optionally having one or more halogen atoms, a C3-C6 alkenyloxy group optionally having one or more halogen atoms, a C3-C6 alkynyloxy group optionally having one or more halogen atoms, and a halogen atom.
Group D: the group consisting of a C1-C6 chain hydrocarbon group optionally having one or more halogen atoms, a C1-C6 alkyloxy group optionally having one or more halogen atoms, a C3-C6 alkenyloxy group optionally having one or more halogen atoms, a C3-C6 alkynyloxy group optionally having one or more halogen atoms, a C1-C6 alkylsulfanyl group optionally having one or more halogen atoms, a C1-C6 alkylsulfinyl group optionally having one or more halogen atoms, a C1-C6 alkylsulfonyl group optionally having one or more halogen atoms, NR¹⁷R¹⁸, C(O)R¹⁷, OC(O)R¹⁷, C(O)OR¹⁷, a cyano group, a nitro group, and a halogen atom.

R¹⁹ represents a C1-C6 chain hydrocarbon group optionally having one or more halogen atoms, or a phenyl group optionally having one or more substituents selected from Group C.
Group C: the group consisting of a C1-C6 chain hydrocarbon group optionally having one or more halogen atoms, a C1-C6 alkyloxy group optionally having one or more halogen atoms, a C3-C6 alkenyloxy group optionally having one or more halogen atoms, a C3-C6 alkynyloxy group optionally having one or more halogen atoms, a C1-C6 alkylsulfanyl group optionally having one or more halogen atoms, a C1-C6 alkylsulfinyl group optionally having one or more halogen atoms, a C1-C6 alkylsulfonyl group optionally having one or more halogen atoms, NR¹⁵R¹⁶, C(O)R¹⁵, OC(O)R¹⁵, C(O)OR¹⁵, a cyano group, a nitro group, and a halogen atom.

The expression "CX-CY" as used herein represents that the number of carbon atom is from X to Y. For example, the expression "C1-C6" represents that the number of carbon atom is from 1 to 6.

The term "halogen atom" represents fluorine atom, chlorine atom, bromine atom, or iodine atom.

The term "chain hydrocarbon group" represents alkyl group, alkenyl group, and alkynyl group.

Examples of the term "alkyl group" include methyl group, ethyl group, propyl group, isopropyl group, 1,1-dimethylpropyl group, 1,2-dimethylpropyl group, 1-ethylpropyl group, butyl group, tert-butyl group (or t-butyl group), pentyl group, hexyl group, heptyl group, octyl group, nonyl group, and decyl group.

Examples of the term "alkenyl group" include vinyl group, 1-propenyl group, 2-propenyl group, 1-methyl-1-propenyl group, 1-methyl-2-propenyl group, 1,2-dimethyl-1-propenyl group, 1,1-dimethyl-2-propenyl group, 1-ethyl-1-propenyl group, 1-ethyl-2-propenyl group, 3-butenyl group, 4-pentenyl group, 5-hexenyl group, heptenyl group, octenyl group, nonenyl group, and decenyl group.

Examples of the term "alkynyl group" includes ethynyl group, 1-propynyl group, 2-propynyl group, 1-methyl-2-propynyl group, 1,1-dimethyl-2-propynyl group, 1-ethyl-2-propynyl group, 2-butynyl group, 4-pentynyl group, 5-hexynyl group, heptynyl group, octynyl group, nonynyl group, and decynyl group.

Examples of the term "alkoxy group" (also referred to as "alkyloxy group" as used herein) include methoxy group, ethoxy group, isopropoxy group, 1,1-dimethylpropoxy group, 1,2-dimethylpropoxy group, 1-ethylpropoxy group, 1-butoxy group, tert-butoxy group, pentoxy group, hexyloxy group, heptyloxy group, octyloxy group, nonyloxy group, decyloxy group, and benzyloxy group.

Examples of the term "alkenyloxy group" include vinyloxy group, 1-propenyloxy group, 2-propenyloxy group, isopropenyloxy group, 1-butenyloxy group, 2-butenyloxy group, 3-butenyloxy group, 1-isobutenyloxy group, 2-isobutenyloxy group, 1-pentenyloxy group, 2-pentenyloxy group, 3-pentenyloxy group, and 4-pentenyloxy group.

Examples of the term "alkynyloxy group" include ethynyloxy group, 1-propynyloxy group, 2-propynyloxy group, 1-butynyloxy group, 2-butynyloxy group, 3-butynyloxy group, 1-pentynyloxy group, 2-pentynyloxy group, 3-pentynyloxy group, 4-pentynyloxy group, 1-hexynyloxy group, 2-hexynyloxy group, and 3-hexynyloxy group.

The term "C2-C10 haloalkyl group" represents a group wherein one or more hydrogen atoms in the C2-C10 alkyl group are substituted with halogen atoms. Examples of the "C2-C10 haloalkyl group" include chloroethyl group, 2,2,2-trifluoroethyl group, 2-bromo-1,1,2,2-tetrafluoroethyl group, 2,2,3,3-tetrafluoropropyl group, 1-methyl-2,2,3,3-tetrafluoropropyl group, perfluorohexyl group, and perfluorodecyl group.

The term "C1-C6 alkyl group optionally having one or more halogen atoms" represents a group wherein one or more hydrogen atoms in the C1-C6 alkyl group may be substituted with halogen atoms, and includes, for example, 2,2,2-trifluoroethyl group, 2,2,3,3-tetrafluoropropyl group, and 2,2,3,4,4,4-hexafluoropropyl group.

The term "C1-C6 alkoxy group optionally substituted with one or more halogen atoms" represents a group wherein one or more hydrogen atoms in the C1-C6 alkoxyl group may be substituted with halogen atoms, and includes, for example, 2,2,2-trifluoroethoxy group, 2,2,3,3-tetrafluoropropoxy group, and 2,2,3,4,4,4-hexafluoropropoxy group.

The term "(C1-C5 alkyloxy)C2-C5 alkyl group having one or more halogen atoms" represents a group wherein the (C1-C5 alkyloxy) and/or (C2-C5 alkyl) have one or more halogen atoms, and includes, for example, 2-(trifluoromethoxy)ethyl group, 2,2-difluoro-3-methoxypropyl group, 2,2-difluoro-3-(2,2,2-trifluoroethoxy)propyl group, and 3-(2-chloroethoxy)propyl group.

The term "(C1-C5 alkylsulfanyl)C2-C5 alkyl group having one or more halogen atoms" represents a group wherein the (C1-C5 alkylsulfanyl) and/or (C2-C5 alkyl) have one or more halogen atoms, and includes, for example, 2,2-difluoro-2-(trifluoromethylthio)ethyl group.

The term "(C1-C5 alkylsulfinyl)C2-C5 alkyl group having one or more halogen atoms" represents a group wherein the (C1-C5 alkylsulfinyl) and/or (C2-C5 alkyl) have one or more halogen atoms, and includes, for example, 2,2-difluoro-2-(trifluoromethanesulfinyl)ethyl group.

The term "(C1-C5 alkylsulfonyl)C2-C5 alkyl group having one or more halogen atoms" represents a group wherein the (C1-C5 alkylsulfonyl) and/or (C2-C5 alkyl) have one or more halogen atoms, and includes, for example, 2,2-difluoro-2-(trifluoromethanesulfonyl)ethyl group.

The term "(C3-C7 cycloalkyl)C1-C3 alkyl group having one or more substituents selected from Group G" represents a group wherein the (C3-C7 cycloalkyl) and/or (C1-C3 alkyl) have one or more substituents selected from Group G, and includes, for example, (2,2-difluorocyclopropyl)methyl group, [1-(trifluoromethyl)cyclopropyl]methyl group, [2-(trifluoromethyl)cyclopropyl]methyl group, 2-cyclopropyl-1,1,2,2-tetrafluoroethyl group, 2-cyclopropyl-3,3,3-trifluoropropyl group, and 1,1,2,2-tetrafluoro-2-[2-(trifluoromethyl)cyclopropyl]ethyl group.

The term "C3-C7 cycloalkyl group having one or more substituents selected from Group G" includes, for example, 2,2-difluorocyclopropyl group, 1-(2,2,2-trifluoroethyl)cyclopropyl group, and 4-trifluoromethyl)cyclohexyl group.

The term "alkylsulfanyl group" represents a sulfanyl group having the above-mentioned alkyl group, and includes, for example, methylsulfanyl group, ethylsulfanyl group, propylsulfanyl group, and isopropylsulfanyl group.

The term "alkylsulfinyl group" represents a sulfinyl group having the above-mentioned alkyl group, and includes, for example, methylsulfinyl group, ethylsulfinyl group, propylsulfinyl group, and isopropylsulfinyl group.

The term "alkylsulfonyl group" represents a sulfonyl group having the above-mentioned alkyl group, and includes, for example, methylsulfonyl group, ethylsulfonyl group, propylsulfonyl group, and isopropylsulfonyl group.

The term "aryl group" includes, for example, phenyl group and naphthyl group.

The term "heteroaryl group" includes, for example, furanyl group, pyrrolyl group, imidazolyl group, oxazolyl group, pyridyl group, pyridazinyl group, pyrimidinyl group, pyrazinyl group, and triazinyl group.

The term "arylsulfonyl" includes, for example, phenylsulfonyl group, 1-naphthylsulfonyl group, and 2-naphthylsulfonyl group.

The term "5 or 6-membered aromatic heterocyclic group" includes, for example, pyrrolyl group, furyl group, thienyl group, pyrazolyl group, imidazolyl group, triazolyl group, tetrazolyl group, oxazolyl group, isoxazolyl group, thiazolyl group, oxadiazolyl group, thiadiazolyl group, pyridyl group, pyridazinyl group, pyrimidinyl group, and pyrazinyl group.

The term "3 or 7-membered non-aromatic heterocyclic group" includes, for example, aziridine ring, azetidine ring, pyrrolidine ring, imidazoline ring, imidazolidine ring, piperidine ring, tetrahydropyrimidine ring, hexahydropyrimidine ring, piperazine ring, azepane ring, oxazolidine ring, isoxazolidine ring, 1,3-oxadinane ring, morpholine ring, 1,4-oxazepane ring, thiazolidine ring, isothiazolidine ring, 1,3-thiazinane ring, thiomorpholine ring, and 1,4-thiazepane ring.

The term "ammonia or an ammonium salt" includes, for example, ammonia gas, ammonia methanol solution, ammonia ethanol solution, ammonia 2-propanol solution, ammonia 1,4-dioxane solution, ammonia tetrahydrofuran solution, ammonium chloride, ammonium acetate, ammonium carbamate, ammonium formate, ammonium hydrogen carbonate, ammonium carbonate, triammonium phosphate, diammonium phosphate, monoammonium citrate, diammonium citrate, triammonium citrate, diammonium succinate, and ammonium amidosulfate.

The term "N-halosuccinimide oxidizing agent" includes, for example, N-bromosuccinimide and N-chlorosuccinimide.

The term "quinone oxidizing agent" includes, for example, p-quinone oxidizing agent and o-quinone oxidizing agent.

The term "R²OH wherein the symbol has the same meaning as defined above" includes, for example, methanol, ethanol, and 2-propanol.

As used herein, "Me" represents a methyl group, "Et" represents an ethyl group, "Bn" represents a benzyl group, and "THF" represents tetrahydrofuran.

The compounds as described herein may have the tautomers as shown below.

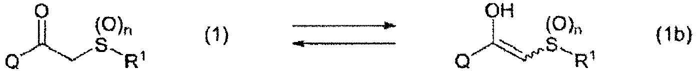

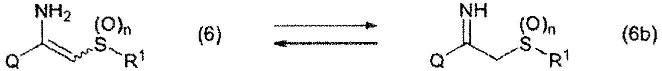

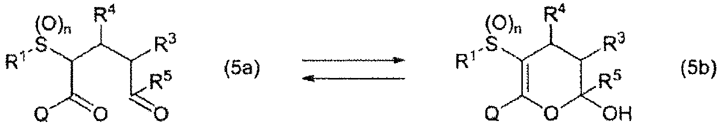

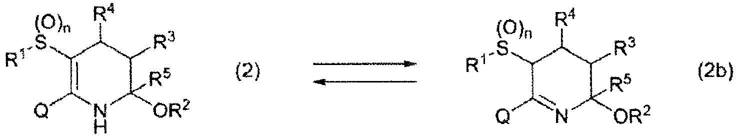

Examples of embodiments of a compound represented by formula (2) include the following compounds.

A compound of formula (2) wherein Q represents a pyridyl group optionally having one or more substituents that are inactivated for an oxidation reaction, a pyrimidinyl group optionally having one or more substituents that are inactivated for an oxidation reaction, or a pyrazinyl group optionally having one or more substituents that are inactivated for an oxidation reaction.

A compound of formula (2) wherein Q represents a pyridyl group optionally having one or more substituents selected from Group I, a pyrimidinyl group optionally having one or more substituents selected from Group I, or a pyrazinyl group optionally having one or more substituents selected from Group I.
Group I: the group consisting of a C1-C6 alkyl group optionally having one or more halogen atoms, a C1-C6 alkylsulfonyl group optionally having one or more halogen atoms, a C6-C10 arylsulfonyl group optionally having one or more C1-C6 alkyl groups, a C6-C10 arylsulfonyl group optionally having one or more nitro groups, OR¹², NR¹³R¹⁴, a cyano group, a nitro group, and a halogen atom.

A compound of formula (2) wherein Q represents a pyridyl group optionally having one or more substituents selected from Group J, a pyrimidinyl group optionally having one or more substituents selected from Group J, or a pyrazinyl group optionally having one or more substituents selected from Group J.
Group J: the group consisting of a C1-C6 alkyl group optionally having one or more halogen atoms, OR¹², a nitro group, and a halogen atom.

A compound of formula (2) wherein Q represents a pyridyl group optionally having one or more substituents selected from Group K, a pyrimidinyl group optionally having one or more substituents selected from Group K, or a pyrazinyl group optionally having one or more substituents selected from Group K.
Group K: the group consisting of OR¹² and a halogen atom.

A compound of formula (2) wherein Q represents a pyridyl group optionally having one or more substituents selected from Group L, a pyrimidinyl group optionally having one or more substituents selected from Group L, or a pyrazinyl group optionally having one or more substituents selected from Group L.
Group L: the group consisting of a C1-C10 alkyloxy group optionally having one or more halogen atoms, and a halogen atom.

A compound of formula (2) wherein R¹ represents a methyl group, an ethyl group, a propyl group, a trifluoromethyl group, or a pentafluoroethyl group.

A compound of formula (2) wherein R² represents a methyl group or an ethyl group.

A compound of formula (2) wherein R³ represents a C1-C6 alkyl group optionally having one or more substituents that are inactivated for an oxidation reaction, OR¹⁰, NR¹⁰R¹¹, NR¹⁰C(O)R¹¹, NR¹⁰C(O)OR¹¹, S(O)_{y}R¹⁰, a halogen atom, or a hydrogen atom.

A compound of formula (2) wherein R³ represents a C1-C6 alkyl group optionally having one or more substituents that are inactivated for an oxidation reaction, OR¹⁰, NR¹⁰R¹¹, NR¹⁰C(O)R¹¹, NR¹⁰C(O)OR¹¹, S(O)_{y}R¹⁰, or a hydrogen atom.

A compound of formula (2) wherein R³ represents OR¹⁰ or a hydrogen atom.

A compound of formula (2) wherein R¹⁰ represents a methyl group, an ethyl group, a propyl group, or a benzyl group.

A compound of formula (2) wherein R¹¹ represents a methyl group, an ethyl group, a propyl group, or a benzyl group.

A compound of formula (2) wherein R⁴ and R⁵ are identical to or different from each other, and represent a C1-C6 alkyl group optionally having one or more halogen atoms.

A compound of formula (2) wherein R⁴ and R⁵ are identical to or different from each other, and represent an aryl group optionally having one or more substituents that are inactivated for an oxidation reaction or a heteroaryl group optionally having one or more substituents that are inactivated for an oxidation reaction.

A compound of formula (2) wherein R⁴ and R⁵ represent a hydrogen atom.

A compound of formula (2) wherein n represents 1 or 2.

A compound of the present compound wherein R¹ is preferably a methyl group, an ethyl group, a propyl group, a trifluoromethyl group, or a pentafluoroethyl group, more preferably an ethyl group.

A compound of the present compound wherein R² is preferably a methyl group, an ethyl group, a propyl group, an isopropyl group, or a benzyl group, more preferably a methyl group or an ethyl group.

A compound of the present compound wherein R³ is preferably a C1-C6 alkyl group optionally having one or more substituents that are inactivated for an oxidation reaction, OR¹⁰, NR¹⁰R¹¹, NR¹⁰C(O)R¹¹, NR¹⁰C(O)OR¹¹, S(O)_{y}R¹⁰, a halogen atom, or a hydrogen atom, more preferably OR¹⁰, NR¹⁰R¹¹, NR¹⁰C(O)R¹¹, NR¹⁰C(O)OR¹¹, S(O)_{y}R¹⁰, or a hydrogen atom, particularly preferably OR¹⁰ or a hydrogen atom.

A compound of the present compound wherein R¹⁰ is preferably a methyl group, an ethyl group, a propyl group, or a benzyl group.

A compound of the present compound wherein R¹¹ is preferably a methyl group, an ethyl group, a propyl group, or a benzyl group.

A compound of the present compound wherein R⁴ and R⁵ are identical to or different from each other, and are preferably a C1-C6 alkyl group optionally having one or more halogen atoms, or a hydrogen atom, more preferably a hydrogen atom.

A compound of the present compound wherein n is preferably 1 or 2, more preferably 2.

The present invention is particularly described as follows.

In the present invention, a compound represented by formula (4) (hereinafter, referred to as "compound (4)") is prepared by oxdizing a compound represented by formula (2) (hereinafter, referred to as "compound (2)") in the presence of an organic oxdizing agent according to the following process.

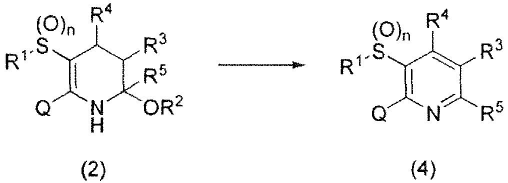

The organic oxdizing agent includes, for example, a p-quinone oxidizing agent represented by formula (7) 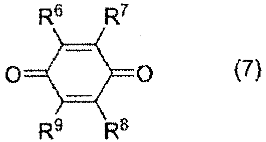 wherein R⁶, R⁷, R⁸, and R⁹ each independently represent a hydrogen atom, a methyl group, a tert-butyl group, a halogen atom, or a cyano group
(hereinafter, referred to as "compound (7)"); an o-quinone oxidizing agent represented by formula (8) 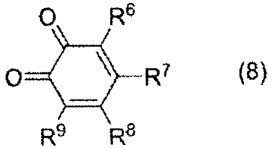 wherein the symbols are the same as those defined above (hereinafter, referred to as "compound (8)"); an N-halosuccinimide oxidizing agent; a hypervalent iodine oxidizing agent such as (dichloroiodo)benzene, (diacetoxyiodo)benzene, [bis(trifluoroacetoxy)iodo]benzene, [hydroxy(tosyloxy)iodo]benzene, 1,1,1-triacetoxy-1,1-dihydro-1,2-benziodoxol-3(1H)-one and 2-iodoxybenzoic acid; an N-oxide compound such as pyridine N-oxide, 4-chloropyridine N-oxide, 4-methylpyridine N-oxide, 2,6-dichloropyridine N-oxide, 2,6-dibromopyridine N-oxide, 4-cyanopyridine N-oxide, 4-methoxypyridine N-oxide, 4-(dimethylamino)pyridine N-oxide, 4-nitropyridine N-oxide, 2-hydroxypyridine N-oxide, isonicotinic acid N-oxide, 4-phenylpyridine N-oxide, 4-nitroquinoline N-oxide, 2,6-lutidine N-oxide, and is preferably the compound (7), the compound (8), or an N-halosuccinimide oxidizing agent, more preferably the compound (7) or the compound (8).

A used amount of the organic oxidizing agent is usually within a range of 1 to 10 molar ratio(s), preferably 1 to 5 molar ratio(s), relative to 1 mole of the compound (2).

The reaction is usually carried out in the presence of a solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene; halogenated hydrocarbon solvents such as dichloromethane and 1,2-dichloroethane; ketone solvents such as acetone and methyl isobutyl ketone; carboxylic acid solvents such as acetic acid and formic acid; water, and mixed solvents thereof.

A used amount of the solvent is usually a range of 1 to 20 time(s) weight, preferably a range of 3 to 20 times weight, relative to a weight of the compound (2).

An acid may be added in order to facilitate the oxidative reaction. Examples of the acid include carboxylic acids such as acetic acid, formic acid, citric acid, oxalic acid, 3-phenylacrylic acid, benzoic acid, 4-chlorobenzoic acid, terephthalic acid, isophthalic acid and 1-naphthalenecarboxylic acid; phosphoric acids; sulfonic acids such as methanesulfonic acid and 4-toluenesulfonic acid monohydrate; hydrogen halides such as hydrogen chloride, hydrogen bromide, hydrogen iodide and hydrogen fluoride; halogenated oxoacids such as hypochlorous acid, chlorous acid, hypobromous acid, bromous acid, hypoiodous acid and iodic acid; sulfuric acid, nitric acid, tetrafluoroboric acid, and chromic acid.

A used amount of the acid is usually a range of 0.01 to 100 molar ratios, preferably a range of 1 to 10 molar ratio(s), relative to 1 mole of the compound (2).

An order of mixing the compound (2) and an organic oxdizing agent includes a process of firstly mixing the compound (2) with the solvent and subsequently adding an organic oxdizing agent to the mixture, and a process of firstly mixing an organic oxidizing agent with the solvent and subsequently adding a crystal of the compound (2) or a solution of the compound (2) to the mixture.

A reaction temperature of the reaction is usually within a range of -20 to 150°C, preferably within a range of 20 to 120°C.

A reaction period of the reaction is usually within a range of 0.5 to 30 hours, preferably within a range of 0.5 to 20 hours.

After the reaction is completed, the reaction mixture can be worked up, for example, the reaction mixture and water are mixed and extracted with an organic solvent, and the resulting organic layer is then dried and concentrated; or the reaction mixture and an aqueous solution of an alkali metal hydroxide such as sodium hydroxide and potassium hydroxide are mixed and extracted with an organic solvent, and the resulting organic layer is then dried and concentrated, to isolate the compound (4). The isolated compound (4) can be further purified by a purification process such as chromatography and recrystallization.

The compound (2) can be prepared, for example, according to the following process. Namely, examples of the process include a process for reacting a compound represented by formula (6) (hereinafter, referred to as "compound (6)") with a compound represented by formula (3) (hereinafter, referred to as "compound (3)"), and a process for reacting a compound represented by formula (5a) (hereinafter, referred to as "compound (5a)") or a compound represented by formula (5b) (hereinafter, referred to as "compound (5b)") with ammonia or an ammonium salt in the presence of an alcohol. 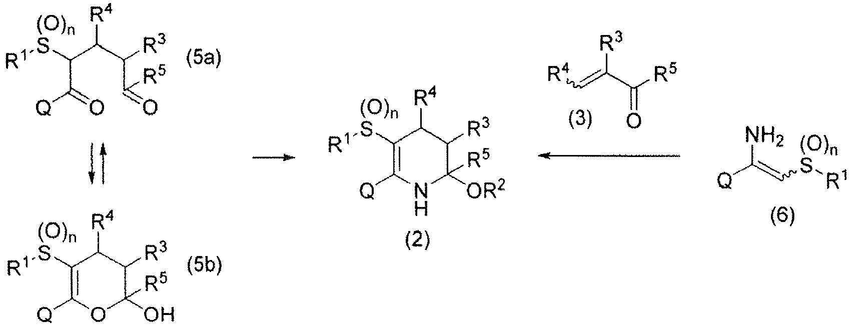 wherein the symbols are the same as those defined above.

Firstly, a process for preparing the compound (2) from the compound (6) and the compound (3) is described.

The compound (2) can be prepared by reacting the compound (6) with the compound (3).

The compound (3) is a commercially available compound, or may be prepared according to the method described in Eur. J. Org. Chem. 2007, 4205-4216.

A used amount of the compound (3) is usually a range of 0.8 to 10 molar ratios, preferably a range of 1 to 5 molar ratio(s), relative to 1 mole of the compound (6).

The reaction is usually carried out in the presence of a solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene; halogenated hydrocarbon solvents such as dichloromethane and 1,2-dichloroethane; ketone solvents such as acetone and methyl isobutyl ketone; carboxylic acid solvents such as formic acid and acetic acid, and mixed solvents thereof.

A used amount of the solvent is usually a range of 1 to 10 time(s) weight, preferably a range of 2 to 8 times weight, relative to a weight of the compound (6).

An acid may be added in order to facilitate the reaction. Examples of the acid include carboxylic acids such as acetic acid, formic acid, citric acid, oxalic acid, 3-phenylacrylic acid, benzoic acid, 4-chlorobenzoic acid, terephthalic acid, isophthalic acid and 1-naphthalenecarboxylic acid; phosphoric acids; sulfonic acids such as methanesulfonic acid and 4-toluenesulfonic acid monohydrate; hydrogen halides such as hydrogen chloride, hydrogen bromide, hydrogen iodide and hydrogen fluoride; halogenated oxoacids such as hypochlorous acid, chlorous acid, hypobromous acid, bromous acid, hypoiodous acid and iodic acid; sulfuric acid, nitric acid, tetrafluoroboric acid, and chromic acid, and are preferably carboxylic acids, more preferably acetic acid.

A used amount of the acid is usually a range of 0.01 to 10 molar ratios, relative to 1 mole of the compound (6).

A reaction temperature of the reaction is usually within a range of -20 to 150°C, preferably within a range of 20 to 120°C.

A reaction period of the reaction is usually within a range of 0.5 to 20 hours, preferably within a range of 0.5 to 12 hours.

After the reaction is completed, the reaction mixture can be worked up, for example, the reaction mixture is concentrated; the reaction mixture and water are mixed and extracted with an organic solvent, and the resulting organic layer is then dried and concentrated; or the reaction mixture and an aqueous solution of an alkali metal hydroxide such as sodium hydroxide and potassium hydroxide are mixed and extracted with an organic solvent, and the resulting organic layer is then dried and concentrated, to isolate the compound (2). The isolated compound (2) can be further purified by a purification process such as chromatography and recrystallization.

The compound (6) can be prepared, for example, by reacting a compound represented by formula (1) (hereinafter, referred to as "compound (1)") with ammonia or an ammonium salt. 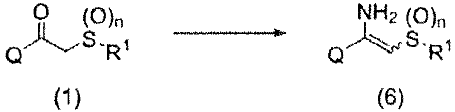 wherein the symbols are the same as those defined above.

A used amount of ammonia or the ammonium salt is usually a range of 1 to 10 molar ratio(s) as a conversion in terms of effective nitrogen atoms, preferably a range of 2 to 5 molar ratios as a conversion in terms of effective nitrogen atoms, relative to 1 mole of the compound (1).

The reaction is usually carried out in the presence of a. solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether, and mixed solvents thereof.

A used amount of the solvent is usually a range of 1 to 10 time(s) weight, preferably a range of 1 to 5 time(s) weight, relative to a weight of the compound (1).

The compound (6) can be prepared, for example, by reacting a compound represented by formula (9) (hereinafter, referred to as "compound (9)") with a compound represented by formula (11) (hereinafter, referred to as "compound (11)") in the presence of a base. 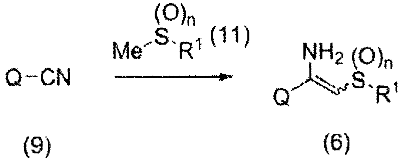 wherein the symbols are the same as those defined above.

The compound (9) is a commercially available compound, or may be prepared according to a known method.

The compound (11) is a commercially available compound, or may be prepared according to the method described in Journal of Molecular Catalysis A: Chemical, 2011, 341(1-2), 57-62.

The reaction is usually carried out in the presence of a solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene, and mixed solvents thereof.

Examples of the base to be used in the reaction include butyllithium, s-butyllithium, t-butyllithium, lithium diisopropylamide, sodium bis(trimethylsilyl)amide, potassium bis(trimethylsilyl)amide, potassium tert-butoxide, sodium methoxide, sodium ethoxide, and alkali metal hydrides such as sodium hydride and potassium hydride.

In the reaction, the compound (11) is usually used in a range of 0.8 to 5 molar ratios, and the base is usually used in a range of 1 to 5 molar ratio(s), relative to 1 mole of the compound (9). Preferably, the compound (11) is used in a range of 1.0 to 1.1 molar ratio(s), and the base is used in a range of 1 to 2 molar ratio(s), relative to 1 mole of the compound (9).

A reaction temperature of the reaction is usually within a range of -78 to 100°C.

A reaction period of the reaction is usually within a range of 0.5 to 24 hours, preferably within a range of 0.5 to 10 hours.

After the reaction is completed, the reaction mixture can be concentrated to isolate the compound (6). The isolated compound (6) can be further purified by a purification process such as recrystallization, chromatography and salting-out.

Next, a process for preparing the compound (2) from the compound (5a) or the compound (5b) is described.

The compound (2) can be prepared by reacting the compound (5a) or the compound (5b) with ammonia or an ammonium salt in the presence of an alcohol.

A used amount of ammonia or the ammonium salt is usually a range of 1 to 10 molar ratio(s) as a conversion in terms of effective nitrogen atoms, preferably a range of 5 to 10 molar ratios as a conversion in terms of effective nitrogen atoms, relative to 1 mole of the compound (5a) or the compound (5b).

Examples of the alcohol include methanol, ethanol, 2-propanol, and mixtures thereof.

A used amount of the alcohol is usually a range of 0.5 to 10 times weight, relative to a weight of the compound (5a) or the compound (5b).

The alcohol can be used as a solvent to react, and the other solvent(s) may be further mixed to react.

Examples of the other solvent(s) include nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene; halogenated hydrocarbon solvents such as dichloromethane and 1,2-dichloroethane; ketone solvents such as acetone and methyl isobutyl ketone; carboxylic acid solvents such as formic acid and acetic acid, and mixed solvents thereof.

A used amount of the other solvent is usually a range of 0.5 to 10 times weight, relative to a weight of the compound (5a) or the compound (5b).

An acid can be added in order to facilitate the reaction, and examples of the acid include carboxylic acids such as acetic acid, formic acid, citric acid, oxalic acid, 3-phenylacrylic acid, benzoic acid, 4-chlorobenzoic acid, terephthalic acid, isophthalic acid and 1-naphthalenecarboxylic acid; phosphoric acids; sulfonic acids such as methanesulfonic acid and 4-toluenesulfonic acid monohydrate; hydrogen halides such as hydrogen chloride, hydrogen bromide, hydrogen iodide and hydrogen fluoride; halogenated oxoacids such as hypochlorous acid, chlorous acid, hypobromous acid, bromous acid, hypoiodous acid and iodic acid; sulfuric acid, nitric acid, tetrafluoroboric acid, and chromic acid.

A used amount of the acid is usually a range of 0.01 to 10 molar ratios, relative to 1 mole of the compound (5a) or the compound (5b).

A reaction temperature of the reaction is usually within a range of -20 to 150°C, preferably within a range of 20 to 120°C.

A reaction period of the reaction is usually within a range of 0.5 to 30 hours, preferably within a range of 0.5 to 25 hours.

After the reaction is completed, the reaction mixture can be worked up, for example, the reaction mixture is concentrated; the reaction mixture and water are mixed and extracted with an organic solvent, and the resulting organic layer is then dried and concentrated; or the reaction mixture and an aqueous solution of an alkali metal hydroxide such as sodium hydroxide and potassium hydroxide are mixed and extracted with an organic solvent, and the resulting organic layer is then dried and concentrated, to isolate the compound (2). The isolated compound (2) can be further purified by a purification process such as chromatography and recrystallization.

The compound (5a) or the compound (5b) can be prepared, for example, according to the following process. Namely, examples of the process include a process for reacting the compound (1) with the compound (3). 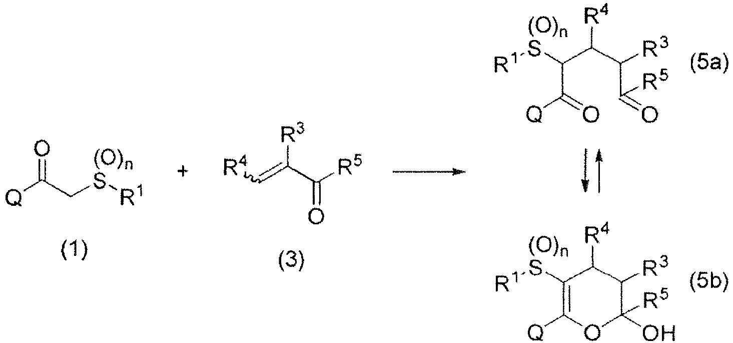 wherein the symbols are the same as those defined above.

The reaction of the compound (1) and the compound (3) can be carried out, for example, in the presence of a base.

Examples of the base include amine bases such as triethylamine, tetramethylethylenediamine, N,N-diisopropylethylamine, 4-(dimethylamino)pyridine, N,N-dimethylaniline, N,N-diethylaniline, 4-methylmorpholine, 1-azabicyclo[2.2.2]octane, 1,4-diazabicyclo[2.2.2]octane, pyridine, 2,6-dimethylpyridine, 1,8-diazabicyclo[5.4.0] undec-7-ene and 1,5-diazabicyclo[4.3.0]non-5-ene; metal alkoxides such as sodium methoxide, sodium ethoxide and potassium tert-butoxide; inorganic bases such as sodium carbonate, sodium hydrogen carbonate, potassium carbonate, sodium hydroxide, potassium hydroxide, calcium hydroxide, barium hydroxide, sodium hydride and potassium hydride.

A used amount of the base is usually a range of 0.01 to 10 molar ratios, preferably a range of 0.01 to 5 molar ratios, relative to 1 mole of the compound (1).

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene; halogenated hydrocarbon solvents such as dichloromethane and 1,2-dichloroethane; ketone solvents such as acetone and methyl isobutyl ketone, and mixed solvents thereof.

A used amount of the solvent is usually a range of 1 to 10 time(s) weight, relative to a weight of the compound (1).

A reaction temperature of the reaction is usually within a range of -20 to 150°C, preferably within a range of 20 to 120°C.

A reaction period of the reaction is usually within a range of 0.5 to 10 hours, preferably within a range of 0.5 to 6 hours.

Although the intermediate compounds prepared in the above processes, i.e., the compound (5a), the compound (5b), the compound (6), and the compound (2) can be isolated according to the above processes, the reaction mixture containing each compound of the intermediates can be used in the next step without isolating the compound. That is, the compound (4) can be prepared from the compound (1) without isolating the intermedicate compounds as indicated below. 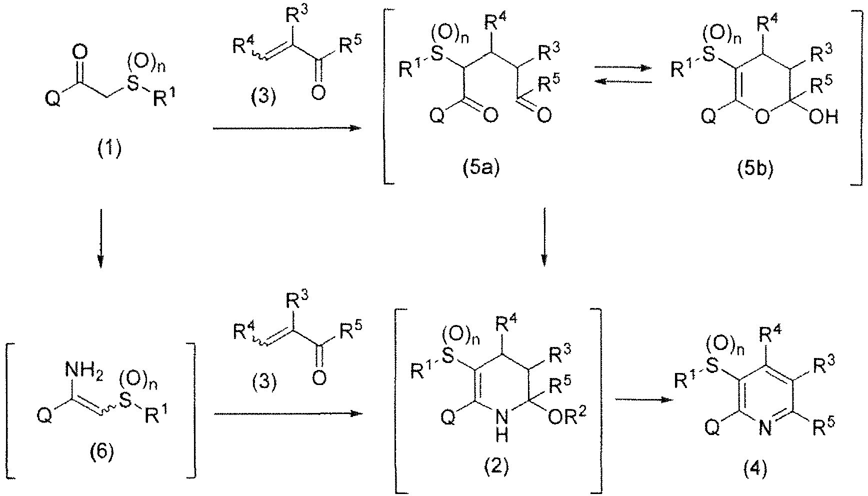 wherein the symbols are the same as those defined above.

The compound (1) can be prepared, for example, by an addition reaction of the compound (9) and the compound (11) in the presence of a base, followed by a hydrolysis reaction of the reaction mixtures. 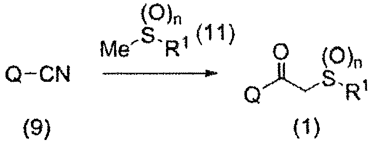 wherein the symbols are the same as those defined above.

The addition reaction is usually carried out in the presence of a solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene, and mixed solvents thereof.

Examples of the base to be used in the addition reaction include butyllithium, s-butyllithium, t-butyllithium, lithium diisopropylamide, sodium bis(trimethylsilyl)amide, potassium bis(trimethylsilyl)amide, potassium tert-butoxide, sodium methoxide, sodium ethoxide, and alkali metal hydrides such as sodium hydride and potassium hydride.

In the addition reaction, the compound (11) is usually used in a range of 0.8 to 5 molar ratios, and the base is usually used in a range of 1 to 5 molar ratio (s), relative to 1 mole of the compound (9). Preferably, the compound (11) is used in a range of 0.8 to 1.1 molar ratios, and the base is used in a range of 1 to 2 molar ratio (s), relative to 1 mole of the compound (9).

A reaction temperature of the reaction is usually within a range of -78 to 100°C, preferably within a range of -78 to 50°C.

A reaction period of the reaction is usually within a range of 0.5 to 24 hours, preferably within a range of 0.5 to 10 hours.

The hydrolysis reaction is usually carried out in the presence of an acid.

Examples of the acid to be used in the hydrolysis reaction include carboxylic acids such as acetic acid, formic acid, citric acid, oxalic acid, 3-phenylacrylic acid, benzoic acid, 4-chlorobenzoic acid, terephthalic acid, isophthalic acid and 1-naphthalenecarboxylic acid; phosphoric acids; sulfonic acids such as methanesulfonic acid and 4-toluenesulfonic acid monohydrate; hydrogen halides such as hydrogen chloride, hydrogen bromide, hydrogen iodide and hydrogen fluoride; halogenated oxoacids such as hypochlorous acid, chlorous acid, hypobromous acid, bromous acid, hypoiodous acid and iodic acid; sulfuric acid, nitric acid, tetrafluoroboric acid, and chromic acid.

In the hydrolysis reaction, the acid is usually used in a range of 3 to 20 molar ratios, preferably a range of 3 to 10 molar ratios, relative to 1 mole of the compound (9).

A reaction temperature of the reaction is usually within a range of 0 to 80°C, preferably within a range of 0 to 50°C.

A reaction period of the reaction is usually within a range of 0.5 to 24 hours, preferably within a range of 0.5 to 10 hours.

After the reaction is completed, the reaction mixture is extracted with an organic solvent and the organic layer can be then worked up such as dried and concentrated to isolate the compound (1). The isolated compound (1) can be further purified by a purification process such as recrystallization, chromatography and salting-out.

In addition, the compound (1) can be prepared, for example, by reacting a compound represented by formula (10) (hereinafter, referred to as "compound (10)") with the compound (11) in the presence of a base. 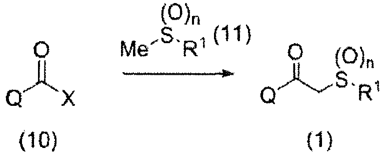 wherein, X represents a C1-C6 alkyloxy group optionally having one or more halogen atoms, a C1-C6 alkylsulfonyloxy group optionally having one or more halogen atoms, a C6-C10 arylsulfonyloxy group optionally having one or more halogen atoms, or a halogen atom, and the other symbols are the same as those defined above.

The compound (10) is a commercially available compound, or may be prepared according to a known method.

The reaction is usually carried out in the presence of a solvent.

Examples of the solvent include alcohol solvents such as methanol, ethanol and 2-propanol; nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene, and mixed solvents thereof.

Examples of the base include butyllithium, s-butyllithium, t-butyllithium, lithium diisopropylamide, sodium bis(trimethylsilyl)amide, potassium bis(trimethylsilyl)amide, potassium tert-butoxide, sodium methoxide, sodium ethoxide, and alkali metal hydrides such as sodium hydride and potassium hydride.

In the reaction, the compound (11) is usually used in a range of 0.8 to 5 molar ratios, and the base is usually used in a range of 1 to 5 molar ratio (s), relative to 1 mole of the compound (10). Preferably, the compound (11) is used in a range of 0.8 to 1.1 molar ratios, and the base is used in a range of 1 to 2 molar ratio(s), relative to 1 mole of the compound (10).

A reaction temperature of the reaction is usually within a range of -78 to 100°C, preferably within a range of -78 to 50°C.

A reaction period of the reaction is usually within a range of 0.5 to 24 hours, preferably within a range of 0.5 to 10 hours.

After the reaction is completed, the reaction mixture is extracted with an organic solvent and the organic layer can be then worked up such as dried and concentrated to isolate the compound (1). The isolated compound (1) can be further purified by a purification process such as recrystallization, chromatography and salting-out.

Further, the compound (1) can be prepared, for example, by the reaction of the compound (10) and a compound represented by formula (15) (hereinafter, referred to as "compound (15)") in the presence of a base, followed by a deacetylation reaction of the resulting reaction mixtures in the presence of an acid. 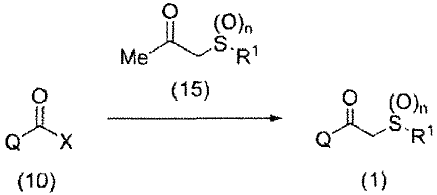 wherein the symbols are the same as those defined above.

The compound (15) is a commercially available compound, or may be prepared according to a known method.

The reaction is usually carried out in the presence of a solvent.

Examples of the solvent include nitrile solvents such as acetonitrile and propionitrile; ether solvents such as diethylether, tetrahydrofuran, methyl t-butyl ether, cyclopentyl methyl ether and ethyleneglycol dimethyl ether; aromatic hydrocarbon solvents such as toluene, xylene and ethylbenzene; halogenated aromatic hydrocarbon solvents such as monochlorobenzene and o-dichlorobenzene, and mixed solvents thereof.

A used amount of the solvent is usually a range of 1 to 20 time(s) weight, preferably a range of 3 to 10 times weight, relative to a weight of the compound (10).

Examples of the base to be used in the reaction of the compound (15) and the compound (10) include amine bases such as triethylamine, tetramethylethylenediamine, N,N-diisopropylethylamine, 4-(dimethylamino)pyridine, N,N-dimethylaniline, N,N-diethylaniline, 4-methylmorpholine, 1-azabicyclo[2.2.2]octane, 1,4-diazabicyclo[2.2.2]octane, pyridine, 2,6-dimethylpyridine, 1,8-diazabicyclo[5.4.0] undec-7-ene and 1,5-diazabicyclo[4.3.0]non-5-ene; metal alkoxides such as sodium methoxide, sodium ethoxide and potassium t-butoxide; inorganic bases such as sodium carbonate, sodium hydrogen carbonate, potassium carbonate, sodium hydroxide, potassium hydroxide, calcium hydroxide, barium hydroxide, sodium hydride and potassium hydride.

A used amount of the base is usually a range of 0.5 to 5 molar ratios, preferably a range of 0.8 to 4 molar ratios, relative to 1 mole of the compound (10).

A used amount of the compound (15) is usually a range of 0.8 to 5 molar ratios, preferably a range of 0.8 to 3 molar ratios, relative to 1 mole of the compound (10).

A Lewis acid may be added in order to facilitate the reaction.

Examples of the Lewis acid include metal halides such as magnesium chloride, scandium chloride and zinc chloride.

A used amount of the Lewis acid is usually a range of 0.5 to 3 molar ratios, preferably a range of 1 to 2 molar ratio(s), relative to 1 mole of the compound (10).

A reaction temperature of the reaction of the compound (15) and the compound (10) is usually within a range of -20 to 70°C, preferably within a range of 0 to 50°C.

A reaction period of the reaction of the compound (15) and the compound (10) is usually within a range of 0.5 to 24 hours, preferably within a range of 0.5 to 10 hours.

Examples of the acid to be used in the deacetylation reaction include carboxylic acids such as acetic acid, formic acid, citric acid, oxalic acid, 3-phenylacrylic acid, benzoic acid, 4-chlorobenzoic acid, terephthalic acid, isophthalic acid and 1-naphthalenecarboxylic acid; phosphoric acids; sulfonic acids such as methanesulfonic acid and 4-toluenesulfonic acid monohydrate; hydrogen halides such as hydrogen chloride, hydrogen bromide, hydrogen iodide and hydrogen fluoride; halogenated oxoacids such as hypochlorous acid, chlorous acid, hypobromous acid, bromous acid, hypoiodous acid and iodic acid; sulfuric acid, nitric acid, tetrafluoroboric acid, and chromic acid.

A used amount of the acid in the deacetylation reaction is usually a range of 1 to 5 molar ratio(s), preferably a range of 1 to 3 molar ratio(s), relative to 1 mole of the compound (10).

A reaction temperature of the deacetylation reaction is usually within a range of -20 to 70°C, preferably within a range of 0 to 50°C.

A reaction period of the deacetylation reaction is usually within a range of 0.5 to 24 hours, preferably within a range of 0.5 to 10 hours.

After the reaction is completed, the reaction mixture is extracted with an organic solvent and the organic layer can be then worked up such as dried and concentrated to isolate the compound (1). The isolated compound (1) can be further purified by a purification process such as recrystallization, chromatography and salting-out.

Hereinafter, the present invention is specifically explained by reciting the examples, however, the present invention should not be limited to the examples.

The "parts" represents "part by weight" unless otherwise specified.

### Example 1

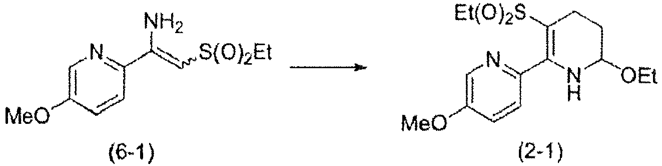

A compound represented by formula (6-1) prepared by reference to WO 2016/121969 10.0 g, ethanol 40 g, and acetic acid 0.25 g were mixed and warmed to 60°C. To the mixture, acrolein 2.8 g was added dropwise, and the mixure was stirred at 60°C for 3 hours. The reaction mixture was concentrated under reduced pressure to give a compound represented by formula (2-1) 13.4 g.

### Compound represented by formula (6-1)

¹H-NMR (CDCl₃, TMS) δ(ppm): 1.40 (3H, t, J=7.6Hz), 3.09 (2H, q, J=7.6Hz), 3.92 (3H, s), 5.26 (1H, s), 6.81 (2H, br), 7.24 (1H, d, J=2.8Hz), 7.64(1H, d, J=8.8Hz), 8.30(1H, d, J=2.8Hz)

### Compound represented by formula (2-1)

¹H-NMR (CDCl₃, TMS) δ (ppm): 1.23 (6H, m), 2.63 (2H, m), 2.92(2H, m), 3.46(1H, m), 3.74(2H, m), 3.88(3H, s), 3.91(1H, d, J=4.0Hz), 4.69(1H, m), 5.16(1H, m), 7.20(1H, dd, J=3.2, 8.8Hz), 7.55(1H, d, J=8.4Hz), 8.25(1H, d, J=2.8Hz)

### Example 2

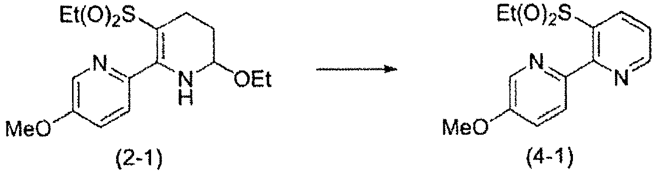

Benzoquinone 0.228 g and acetic acid 2 mL were mixed and warmed to 60°C. To the mixture, a compound represented by formula (2-1) 0.60 g was added into three divided portions over 30 minutes. To the mixture, acetic acid 2 mL was added, and the mixture was stirred at 60°C for 5 hours. The obtained mixture was analyzed with a high-performance liquid chromatography, and then it was confirmed that a compound represented by formula (4-1) was obtained in a yield of 78%.

### Example 3

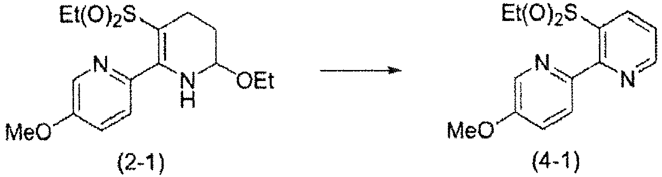

A compound represented by formula (2-1) 0.1 g, chloranil 0.08 g, and acetic acid 1.0 g were mixed and stirred at 20°C for 3 hours, and the mixture was then warmed to 60°C and stirred for another 2 hours. The obtained mixture was analyzed with a high-performance liquid chromatograpy, and then it was confirmed that a compound represented by formula (4-1) was produced at 93% in area percentage.

### Example 4

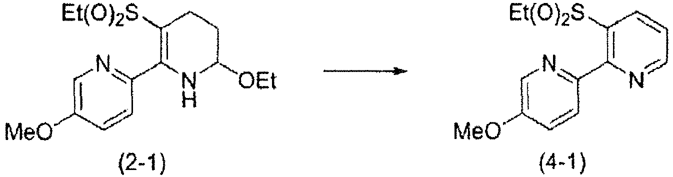

A compound represented by formula (2-1) 0.05 g, benzoquinone 0.02 g, 4-toluenesulfonic acid monohydrate 1.5 mg, and toluene 0.50 g were mixed and stirred at 20°C for 2 hours. The obtained mixture was analyzed with a high-performance liquid chromatography, and then it was confirmed that a compound represented by formula (4-1) was produced at 90% in area percentage.

### Example 5

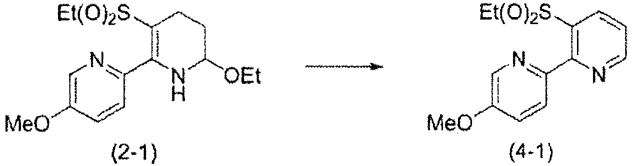

A compound represented by formula (2-1) 0.05 g, N-bromosuccinimide 0.03 g, and acetic acid 0.5 g were mixed and stirred at 20°C for 5 hours, and then warmed to 60°C and stirred for another 4 hours. The obtained mixture was analyzed with a high-performance liquid chromatography, and then it was confirmed that a compound represented by formula (4-1) was produced.

### Example 6

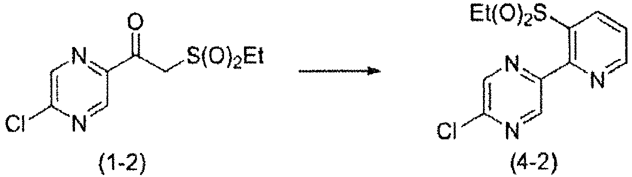

A compound represented by formula (1-2) 1.0 g, toluene 1.0 g, methanol 1.0 g, triethylamine 0.02 g, and acrolein 0.24 g were mixed and stirred at 20°C for 3 hours. To the mixture, ammonium acetate 0.33 g was added and stirred at 20°C for another 15 hours. To the mixture, benzoquinone 0.78 g and acetic acid 3.0 g were added, and then the mixure was warmed to 60°C and stirred for 6 hours. The mixture was cooled to 20°C and concentrated under reduced pressure to obtain a crude product. To the crude product, methyl isobutyl ketone 7.0 g, water 6.0 g, and 24% sodium hydroxide solution 3.6 g were added and portioned to remove an aqueous layer. To the resulting organic layer, 24% sodium hydroxide solution 1.78 g was added and portioned to remove an aqueous layer. The resulting organic layer was washed with water 3.0 g twice and then concentrated to dryness to give a compound represented by formula (4-2) 0.8 g.

### Compound represented by formula (1-2)

¹H-NMR (CDCl₃, TMS) δ(ppm): 1.48(3H, t, J=7.6Hz), 3.28(2H, q, J=7.6Hz), 4.89(2H, s), 8.70(1H, d, J=1.2Hz), 9.08(1H, d, J=1.2Hz)

### Example 7

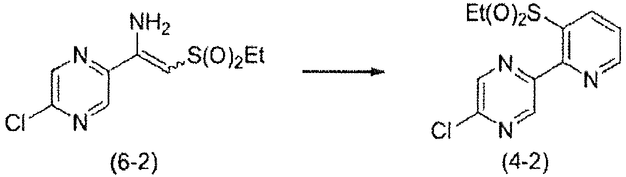

A compound represented by formula (6-2) 0.1 g, benzoquinone 0.05 g, tetrahydrofuran 1 g, acetic acid 0.02 g, and acrolein 0.03 g were mixed at 20°C, and the mixture was then warmed to 40°C and stirred for 4 hours. The mixture was then warmed to 60°C and stirred for another 11 hours. The obtained mixture was analyzed with a high-peformance liquid chromatography and then it was confirmed that a compound represented by formula (4-2) was produced at 70% in area percentage.

### Compound represented by formula (6-2)

¹H-NMR (CDCl₃, TMS) δ(ppm): 1.42 (3H, t, J=7.6Hz), 3.12 (2H, q, J=7.6Hz), 5.44 (1H, s), 6.68 (2H, br), 8.62 (1H, d, J=1.2Hz), 8.76 (1H, d, J=1.6Hz)

### Example 8

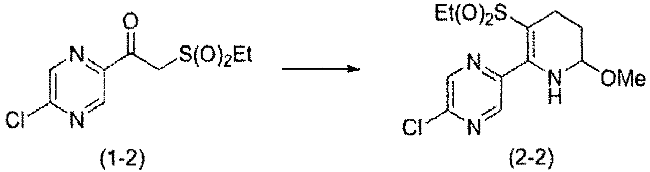

A compound represented by formula (1-2) 5.0 g, toluene 12.5 g, methanol 12.5 g, triethylamine 0.4 g, and acrolein 1.4 g were mixed and stirred at 20°C for one hour. To the mixture, ammonium acetate 1.9 g was added, and the mixture was stirred at 20°C for another 7 hours. To the mixture, water 10 g and ethyl acetate 25 g were added and portioned to remove an aqueous layer. The resulting organic layer was washed with water 10 g and concentrated under reduced pressure to give a compound represented by formula (2-2) 6.0 g.

### Compound represented by formula (2-2)

¹H-NMR (CDCl₃, TMS) δ(ppm): 1.22(3H, t, J=7.2Hz), 1.77(1H, ddt, J=2.4, 5.6, 13.2Hz), 2.22(1H, ddd, J=1.6, 4.4, 12.4Hz), 2.53(1H, ddd, J=2.8, 5.6, 16.0Hz), 2.69(1H, ddd, J=2.0, 5.6, 16.0Hz), 2.96(2H, m), 3.41(3H, s), 4.60(1H, dd, J=2.8, 6.4Hz), 5.12(1H, br s), 8.54(2H, m)

### Example 9

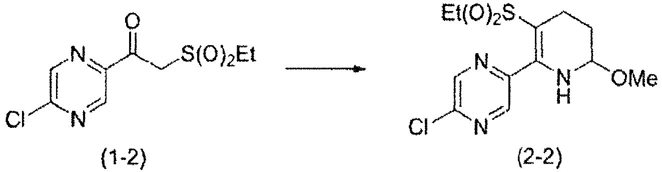

A compound represented by formula (1-2) 0.2 g, methanol 1.0 g, and ammonium acetate 0.3 g were mixed and stirred at 20°C for 2 hours, and the mixture was then warmed to 40°C and stirred for 10 hours. To the mixture, acetic acid 0.005 g and acrolein 0.2 g were added, and the mixture was warmed to 60°C and stirred for 6 hours. The obtained mixture was analyzed with a high-performance liquid chromatography, and then it was confirmed that a compound represented by formula (2-2) was produced at 71% in area percentage.

### Example 10

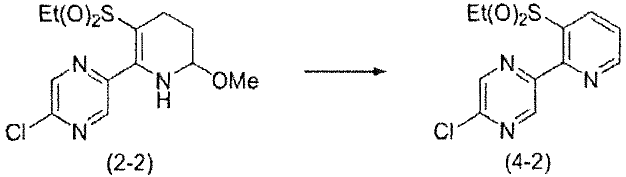

A compound represented by formula (2-2) 3.5 g, benzoquinone 1.3 g, methyl isobutyl ketone 7.0 g, and acetic acid 3.5 g were mixed and stirred at 60°C for 11 hours. After the mixture was cooled to 20°C, methyl isobutyl ketone 21 g, water 21 g, and 24% sodium hydroxide solution 9.7 g were added thereto and portioned to remove an aqueous layer. The resulting organic layer was washed with water 7 g and 24% sodium hydroxide solution 2.8 g once and washed with water 7 g twice, and then concentrated to dryness to obtain a crude product of a compound represented by formula (4-2). The crude product was recrystallized from methyl isobutyl ketone 7.0 g and heptane 14 g, and the obtained precipitation was then filtrated and dried under reduced pressure to give a crystal of the compound represented by formula (4-2) 2.6 g.

### Example 11

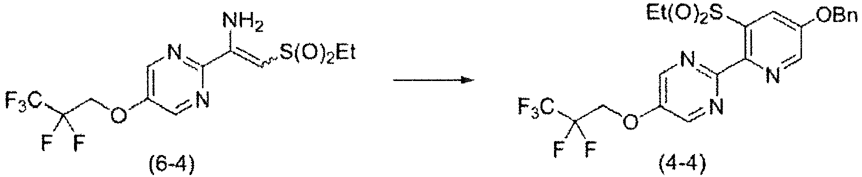

2-(benzyloxy)-2-propenal prepared according to the process described in Eur. J. Org. Chem. 2007, 4205-4216 4.2 g, a compound represented by formula (6-4) 6.5 g, acetic acid 52 µL, and methanol 10 mL were mixed, and the mixture was then warmed to 70°C and stirred for 12 hours. The mixture was concentrated under reduced pressure, and to the obtained concentrate were added 2,3-dichloro-5,6-dicyano-1,4-benzoquinone 4.5 g and toluene 40 mL, and then the mixture was warmed to 100°C and stirred for 5 hours. To the resulting reaction mixture, water was added, and the mixture was extracted with ethyl acetate. The resulting organic layer was dried over anhydrous sodium sulfate and concentrated under reduced pressure. The obtained residue was subjected to a silica gel column chromatography to give a compound represented by formula (4-4) 4.0 g.

### Compound represented by formula (6-4)

¹H-NMR (CDCl₃, TMS) δ(ppm): 1.40 (3H, t, J=7.4Hz), 3.10 (2H, q, J=7.4Hz), 4.62 (2H, t, J=11.8Hz), 6.11 (1H, s), 6.68 (2H, br), 8.52 (2H, s)

### Compound represented by formula (4-4)

¹H-NMR (CDCl₃, TMS) δ(ppm): 1.31 (3H, t, J=7.4Hz), 3.79 (2H, q, J=7.5Hz), 4.60 (2H, t, J=11.8Hz), 5.26 (2H, s), 7.52-7.33 (5H, m), 7.96 (1H, d, J=2.7Hz), 8.57 (2H, s), 8.67 (1H, d, J=2.7Hz)

### Example 12

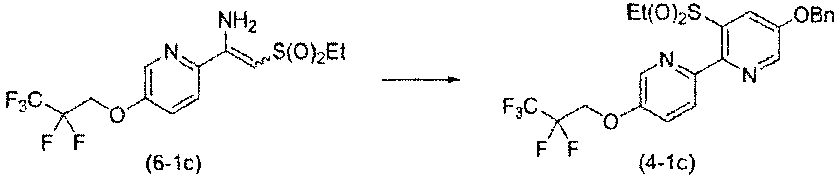

2-(benzyloxy)-2-propenal 1.0 g, a compound represented by formula (6-1c) 1.85 g, acetic acid 29.4 µL, and methanol 2 mL were mixed, and the mixture was then warmed to 60°C and stirred for 6 hours. The mixture was concentrated under reduced pressure, and to the obtained concentrate were added 2,3-dichloro-5,6-dicyano-1,4-benzoquinone 1.28 g and toluene 20 mL, and then the mixture was warmed to 100°C and stirred for 2 hours. The obtained mixture was concentrated under reduced pressure and purified by a silica gel column chromatography to give a compound represented by formula (4-1c) 900 mg.

### Compound represented by formula (4-1c)

¹H-NMR (CDCl₃, TMS) δ(ppm): 1.32 (3H, t, J=7.2Hz), 3.86 (2H, q, J=7.2Hz), 4.52 (2H, dt, J=1.2, 12.4Hz), 5.25 (2H, s), 7.42 (6H, m), 7.79 (1H, dd, J=0.8, 8.8Hz), 8.03 (1H, d, J=2.4Hz), 8.33 (1H, dd, J=0.4, 2.8Hz), 8.59 (1H, d, J=2.8Hz)

### Example 13

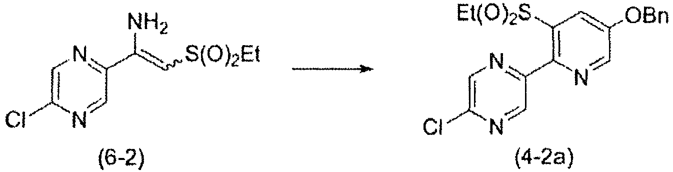

2-(benzyloxy)-2-propenal 9.7 g, a compound represented by formula (6-2) 13.5 g, acetic acid 300 µL, and methanol 60 mL were mixed, and the mixture was then warmed to 70°C and stirred for 14 hours. The mixture was concentrated under reduced pressure, and to the obtained concentrate were added 2,3-dichloro-5,6-dicyano-1,4-benzoquinone 22.3 g and tetrahydrofuran 100 mL, and then the mixture was warmed to 60°C and stirred for 4 hours. After the obtained mixture was diluted with ethyl acetate, the mixture was filtrated through CELITE (registered trademark) and the filtrate was purified by a silica gel column chromatography to give a compound represented by formula (4-2a) 6.0 g.

### Compound represented by formula (4-2a)

¹H-NMR (CDCl₃, TMS) δ(ppm): 1.32 (3H, t, J=7.6Hz), 3.80 (2H, q, J=7.6Hz), 5.27 (2H, s), 7.41 (5H, m), 8.03 (1H, d, J=2.8Hz), 8.55 (1H, s), 8.63 (1H, d, J=2.4Hz), 8.84 (1H, d, J=1.2Hz)

### Example 14

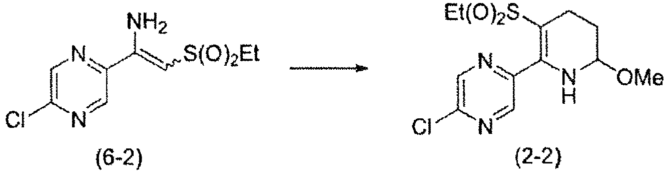

A compound represented by formula (6-2) 13.0 g, methanol 39.0 g, and acetic acid 0.3 g were mixed and warmed to 60°C. To the mixture, acrolein 3.8 g was added dropwise, and the mixture was stirred at 60°C for 8 hours. The mixture was cooled to 20°C and then evaporated to remove the solvent and obtain a crude product of a compound represented by formula (2-2) 20.4 g. The crude product was recrystallized from ethyl acetate 9.3 g, and then the precipitation was filtrated and dried under reduced pressure to give the compound represented by formula (2-2) 12.7 g.

### Example 15

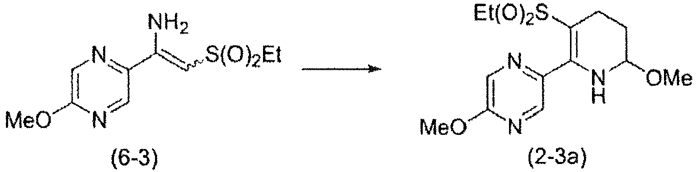

A compound represented by formula (6-3) 5.00 g, methanol 15.00 g, and acetic acid 0.12 g were mixed and warmed to 60°C. To the mixture, acrolein 1.38 g was added dropwise over 35 minutes, and the mixture was stirred at 60°C for 3 hours. The mixture was cooled to 20°C and then concentrated under reduced pressure to remove the solvent and give a compound represented by formula (2-3a) 6.5 g.

### Compound represented by formula (6-3)

¹H-NMR (CDCl₃, TMS) δ(ppm): 1.41(3H, t, J=7.2Hz), 3.11 (2H, q, J=7.2Hz), 4.03 (3H, s), 5.31 (1H, s), 6.69 (2H, br), 8.21 (1H, d, J=1.2Hz), 8.52 (1H, d, J=1.2Hz)

### Compound represented by formula (2-3a)

¹H-NMR (CDCl₃, TMS) δ(ppm): 1.22 (3H, t, J=7.6Hz), 2.53 (1H, dd, J=2.8, 5.2Hz), 2.70(1H, dd, J=2.0, 5.2Hz), 2.95(2H, m), 3.40(3H, s), 3.49(1H, s), 4.00(3H, s), 4.04(1H, dd, J=3.2, 11.2Hz), 4.59(1H, m), 5.17(1H, br s), 8.15(1H, d, J=1.6Hz), 8.32(1H, d, J=1.6Hz)

### Example 16

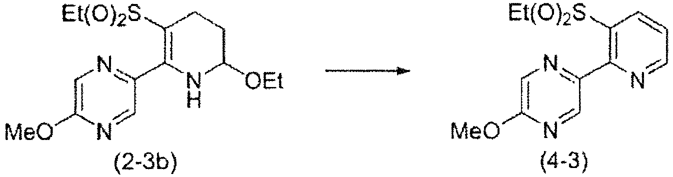

A compound represented by formula (2-3b) 1.00 g, benzoquinone 0.38 g, and toluene 10.00 g were mixed, and the mixture was then warmed to 100°C and stirred for 6 hours. The mixture was cooled to 40°C, and thereto was added acetone 10.00 g. After the obtained mixture was cooled to 30°C, the mixture was mixed with water 10.00 g and 48% potassium hydroxide solution 0.37 g and stirred. The mixture was warmed to 40°C and portioned to remove an aqueous layer and obtain an organic layer 14.32 g. The organic layer was analyzed with a high-performance liquid chromatography and then it was confirmed that a compound represented by formula (4-3) was obtained in a yield of 84%.

### Reference Example 1

To a mixture of 48% sodium hydroxide solution 79.3 g and water 280 g, chloroacetone 80.0 g and ethanethiol 59.1 g were added dropwise at 20°C, and the mixture was warmed to 10°C to 20°C and then stirred for one hour. The mixture was extracted with methyl t-butyl ether 160 g, and the resulting organic layer was washed with water 100 g and then concentrated to give a crude product containing 1-(ethylthio)-2-propanone 104.9 g. The crude product, sodium tungstate 5.7 g, and water 306.6 g were mixed under ice bath and to the stirring mixture was added dropwise 35% hydrogen peroxide 184.8 g, and the mixture was then stirred at 20°C for 3 hours. To the mixture was added 22% sodium sulfite solution 118.9 g, and the mixture was stirred at 20°C for 30 minutes, and sodium chloride 120 g was then added thereto. The mixture was extracted with ethyl acetate 408.8 g five times and the combined organic layer was concentrated. The obtained concentrate was diluted with toluene, and sodium sulfate was added thereto and the mixture was dried. The mixture was filtrated to remove sodium sulfate and concentrated under reduced pressure to remove the organic solvent and give 1-(ethanesulfonyl)-2-propanone 110.3 g.

### Reference Example 2

Sodium sulfite 58 parts and sodium hydrogen carbonate 44 parts are stirred in water 150 parts at 20°C, and an internal temperature of the mixture is then warmed to 50°C. To the mixture, ethanesulfonyl chloride 34 parts is added dropwise over 45 minutes or more, and the mixture is stirred at 50°C for 5 hours and then thereto is added dropwise concentrated sulfuric acid 5.1 parts. After the mixture is stirred at 50°C for one hour, the mixture is warmed to 70°C and thereto is added dropwise chloroacetone 22.6 parts over 10 minutes or more. The obtained mixture is stirred at 70°C for 12 hours and extracted with ethyl acetate at 50°C, and the resulting organic layer is then concentrated to give 1-(ethanesulfonyl)-2-propanone.

### Reference Example 3

Magnesium chloride 19.4 g and tetrahydrofuran 45 g were mixed at 20°C, and thereto was added dropwise triethylamine 34.3 g at 20°C over 20 minutes. To the obtained mixture, a mixture of 1-(ethanesulfonyl)-2-propanone 30.6 g and tetrahydrofuran 15 g was added dropwise at 20°C over 40 minutes, and the mixture was stirred at 20°C for one hour. To the obtained mixture, a mixture of 5-chloro-2-pyrazinecarboxylic acid chloride prepared by reference to WO 2010/103438 30.0 g and toluene 60 g was added dropwise at 20°C over 30 minutes, and the mixture was stirred at 20°C for 2 hours. After the obtained mixture was cooled to 0°C, thereto was added dropwise a mixture of 35% hydrochloric acid 35.3 g and water 60 g at 0°C over 20 minutes, and thereto was then added a mixture of toluene 60 g, water 30 g, and tetrahydrofuran 60 g, and the mixture was stirred at 20°C for 24 hours. The mixture was portioned to remove an aqueous layer, and the resulting organic layer was washed with 5% sodium hydrogen carbonate solution 60 g once and water 60 g once. The organic layer was filtrated to remove an insoluble material, and the filtrate was concentrated to dryness to give a crude product of a compound represented by formula (1-2) 41.5 g.

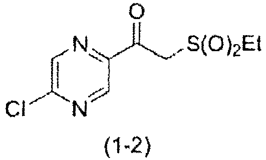

To the crude product, toluene 30 g was added, and the mixture was then warmed to 70°C and kept at the same temperature for 30 minutes to completely dissolve. The resulting solution was cooled to 0°C and kept at the same temperature for 1.5 hours, and then filtrated to obtain the resulting crystal. The crystal was washed with toluene being cooled to 0°C 30 g and dried under reduced pressure to give the compound represented by formula (1-2) 33.0 g.

### Reference Example 4

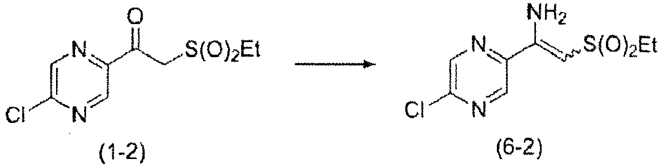

A compound represented by formula (1-2) 6 g, ammonium acetate 9.3 g, and methanol 18 g were mixed at 20°C, and the mixture was then warmed to 70°C and stirred for 2 hours. After the obtained mixture was cooled to 20°C, thereto were added ethyl acetate 39 g and water 20 g, and the mixture was then extracted. The resulting organic layer was concentrated under reduced pressure to give a compound represented by formula (6-2) 5.7 g.

### Reference Example 5

A mixture of 5-chloro-2-cyanopyrimidine 13 g, sodium hydride (60%, oily) 4.6 g, 2,2,3,3,3-pentafluoro-1-propanol 12 mL, and DMF 96 mL was stirred at 20°C for one day. To the resulting reaction mixture, water was added, and the mixture was then extracted with ethyl acetate. The resulting organic layer was dried over anhydrous sodium sulfate and concentrated under reduced pressure. The obtained residue was subjected to a silica gel column chromatography to give a compound represented by formula (9-4) 11 g.

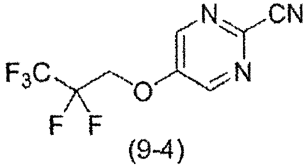

### Compound represented by formula (9-4)

¹H-NMR (CDCl₃, TMS) δ(ppm): 4.65 (2H, t, J=11.7Hz), 8.57 (2H, s)

### Reference Example 6

To a mixture of ethyl methyl sulfone 5.8 g, and THF 90 mL, 2.6 M butyllithium hexane solution 20 mL was added under ice-cooling. The mixture was stirred under ice-cooling for one hour. To the resulting reaction mixture, a mixture of a compound represented by formula (9-4) 11 g and THF 30 mL was added dropwise under ice-cooling, and the mixture was then warmed to 20°C and stirred for 5 hours. To the obtained mixture, 1N hydrochloric acid 100 mL was added, and the mixture was then stirred for 2 hours. The reaction mixture was extracted with ethyl acetate. The resulting organic layer was dried over anhydrous sodium sulfate and concentrated under reduced pressure. The obtained residue was subjected to a silica gel column chromatography to give a compound represented by formula (1-4) 8.0 g.

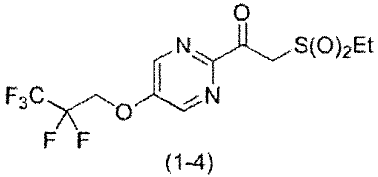

### Compound represented by formula (1-4)

¹H-NMR (CDCl₃, TMS) δ(ppm): 1.51-1.37 (3H, m), 3.30 (2H, q, J=7.5Hz), 4.68 (2H, t, J=11.8Hz), 4.98 (2H, s), 8.67 (2H, s)

### Reference Example 7

A mixture of a compound represented by formula (1-4) 7.8 g, ammonium acetate 8.3 g, and methanol 22 mL was heated to stir at 60°C for 10 hours. The resulting reaction mixture was concentrated under reduced pressure, thereto was added water, and the mixture was then extracted with ethyl acetate. The resulting organic layer was dried over anhydrous sodium sulfate and concentrated under reduced pressure to give a compound represented by formula (6-4) 6.5 g.

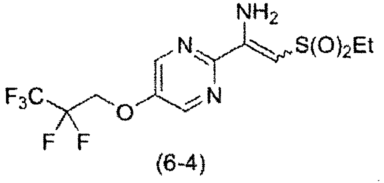

### Comparative Example 1

When a compound represented by formula (2-1) was oxidized according to the method described in Example 2 except using the inorganic oxidizing agents, additives, solvents, and reaction temperatures shown in [Table 1], the result that obtained a compound represented by formula (4-1) (hereinafter, referred to as "compound (4-1)") is shown in [Table 1].

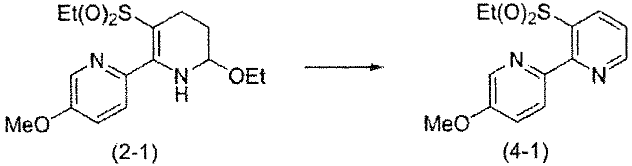

**[Table 1]**

| Oxidizing agent | Condition | | | Compound (4-1) in area percentage |
|---|---|---|---|---|
| | Additive | Solvent | Reaction temperature | |
| Hydrogen peroxide | Sulfuric acid (catalytic amount) | Mixed solvent of acetic acid and water | Room temperature | 4% |
| Nitric acid | None | Acetic acid | 60°C | 28% |
| Sodium hypochlorite | None | Ethanol | Room temperature | 7% |
| Iron(III) chloride | None | Acetic acid | 60°C | 28% |
| Copper(II) chloride | None | Acetic acid | 60°C | 10% |
| Oxygen | None | Acetic acid | 60°C | 17% |

### Comparative Example 2, Comparative Example 3, and Example 17

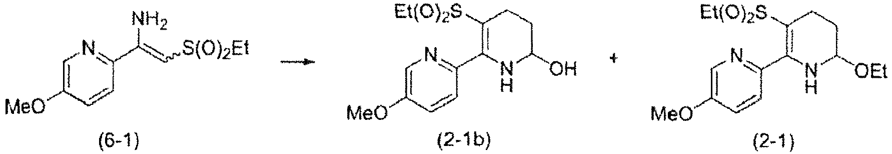

When a compound represented by formula (6-1) was reacted with acrolein according to the process described in Example 1 except using the solvents, acids, and reaction temperatures shown in [Table 2], the reslut that obtained a compound represented by formula (2-1b) (hereinafter, referred to as "compound (2-1b)") or a compound represented by formula (2-1) (hereinafter, referred to as "compound (2-1)") is shown in [Table 2].

**[Table 2]**

| Example No. | Sovent | Acid | Reaction temperature | Compound (2-1b) in area percentage | Compound (2-1) in area percentage |
|---|---|---|---|---|---|
| Comparative Example 2 | THF | None | 60°C | 48% | - |
| Comparative Example 3 | THF | Acetic acid | 60°C | 37% | - |
| Example 17 | Ethanol | None | 100°C (sealed) | - | 75% |
| Example 1 | Ethanol | Acetic acid | 60°C | - | 94% |

### Comparative Example 4

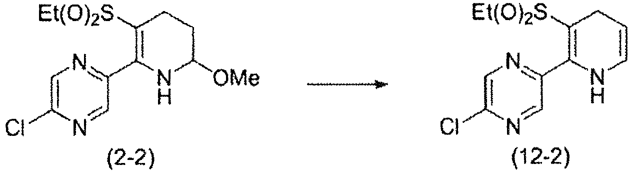

A compound represented by formula (2-2) (hereinafter, referred to as "compound (2-2)") 0.1 g was dissolved in acetic acid 0.06 g and toluene 0.5 g at 20°C, and the mixture was then warmed to 60°C and stirred for 6 hours. A sample was taken after 1 hour, 3 hours, and 6 hours from the start of a stirring at 60°C and then analyzed with a high-performance liquid chromatography. The result of the analysis is shown in [Table 3].

**[Table 3]**

| Reaction period | Compound (2-2) in area percentage | Compound (12-2) in area percentage |
|---|---|---|
| 1 hour | 62% | 19% |
| 3 hours | 35% | 42% |
| 6 hours | 39% | 15% |

It was confirmed that a compound represented by formula (12-2) (hereinafter, referred to as "compound (12-2)") was produced at only low yield. Further, it was also confirmed that the reaction proceeded slowly and thus a lot of by-products containing tar products were generated due to the extended reaction period because the obtained compound (12-2) was unstable.

The mixture was stirred at 60°C for 6 hours and cooled to 20°C, and then thereto was added water and the mixture was portioned. When the resulting organic layer was concentrated under reduced pressure, the dimerization of the compound (12-2) proceeded, and thus the compound (12-2) could not be isolated.

### Comparative Example 5

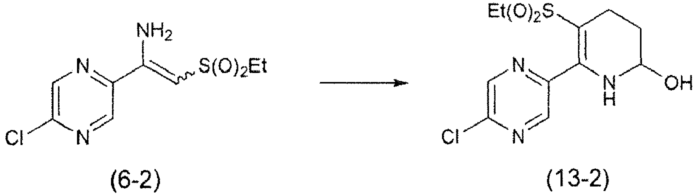

A compound represented by formula (6-2) 0.1 g, piperidine 0.01 g, and acrolein 0.03 g were dissolved in tetrahydrofuran 1 g, and the mixture was then warmed to 60°C and stirred for 4 hours. To facilitate the reaction, to the mixture was further added acrolein 0.05 g, and the mixture was stirred at 60°C for another 5 hours. The mixture was analyzed with a high-performance liquid chromatography, and then it was confirmed that a compound represented by formula (13-2) (hereinafter, referred to as "compound (13-2)") was produced at 67% in area percentage. Meanwhile, NMR indicated that the compound (13-2) was degradated and complicated in the concentration condition under reduced pressure (10 mbar, water bath temperature 30°C, 10 minutes), and thus the compound (13-2) was given as some complex mixtures.

### Industrial Applicability

A method of the present invention allows preparing a pyridine compound useful as a pesticide or its preparation intermediate.

## Claims

1. A method for preparing a compound represented by formula (4) : 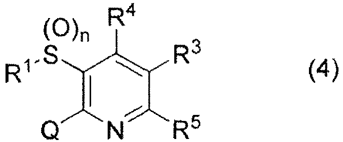 wherein,
Q represents a pyridyl group, a pyrimidinyl group, a pyrazinyl group, or a pyridazinyl group, wherein the pyridyl group, the pyrimidinyl group, the pyrazinyl group and the pyridazinyl group each independently may have optionally one or more substituents that are inactivated for an oxidation reaction;
R¹ represents a C1-C6 alkyl group optionally having one or more halogen atoms;
R³ represents a C1-C6 alkyl group, an aryl group, a heteroaryl group, wherein the C1-C6 alkyl group, the aryl group and the heteroaryl group each independently may have optionally one or more substituents that are inactivated for an oxidation reaction, OR¹⁰, NR¹⁰R¹¹, NR¹⁰OR¹¹, S(O)_{y}R¹⁰, NR¹⁰C(O)R¹¹, NR¹⁰C(O)OR¹¹, a halogen atom, or a hydrogen atom;
R⁴ and R⁵ are identical to or different from each other and represent a C1-C6 alkyl group optionally having one or more halogen atoms, an aryl group, a heteroaryl group, wherein the aryl group and the heteroaryl group each independently may have optionally one or more substituents that are inactivated for an oxidation reaction, or a hydrogen atom;
R¹⁰ and R¹¹ are identical to or different from each other and represent a C1-C6 alkyl group optionally having one or more substituents that are inactivated for an oxidation reaction; and
n and y each independently represent 0, 1, or 2, which comprises a step of reacting a compound represented by formula (2): 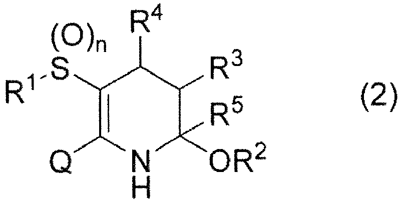
wherein, R² represents a C1-C6 alkyl group optionally having one or more halogen atoms, and the other symbols have the same meanings as defined above,
with an organic oxidizing agent selected from the group consisting of a quinone oxidizing agent, an N-halosuccinimide oxidizing agent, a hypervalent iodine oxidizing agent, and an N-oxide compound.

2. The method according to claim 1, wherein R³ represents OR¹⁰, NR¹⁰R¹¹, NR¹⁰OR¹¹, S(O)_{y}R¹⁰, or a hydrogen atom, and R⁴ and R⁵ represent a hydrogen atom.

3. The method according to claim 1, wherein R¹ represents an ethyl group, R² represents a C1-C6 alkyl group, R³ represents a hydrogen atom or a benzyloxy group, R⁴ and R⁵ represent a hydrogen atom, n represents 2, and Q represents a pyridyl group, a pyrimidinyl group, a pyrazinyl group, or a pyridazinyl group, wherein the pyridyl group, the pyrimidinyl group, the pyrazinyl group and the pyridazinyl group each independently may have optionally a halogen atom or a C1-C6 alkoxy group optionally substituted with a halogen atom.

4. The method according to any one of claims 1 to 3, wherein the organic oxidizing agent is a p-quinone oxidizing agent represented by formula (7): 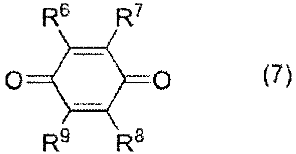 wherein, R⁶, R⁷, R⁸, and R⁹ each independently represent a hydrogen atom, a methyl group, a t-butyl group, a halogen atom, or cyano group,
an o-quinone oxidizing agent represented by formula (8): 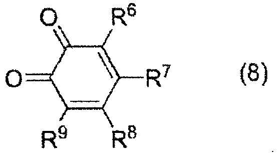 wherein the symbols have the same meanings as defined above, or an N-halosuccinimide oxidizing agent.

5. The method according to any one of claims 1 to 4, which comprises a step of reacting a compound represented by formula (6): 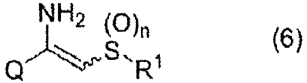 wherein the symbols have the same meanings as defined above, with a compound represented by formula (3): 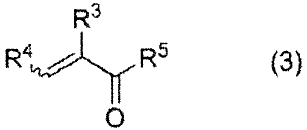 wherein the symbols have the same meanings as defined above, under the presence of an alcohol represented by
R²OH
wherein the symbols have the same meanings as defined above, to obtain the compound represented by formula (2).

6. The method according to any one of claims 1 to 4, which comprises a step of reacting a compound represented by formula (5a): 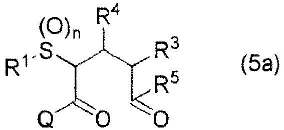 wherein the symbols have the same meanings as defined above, or a compound represented by formula (5b): 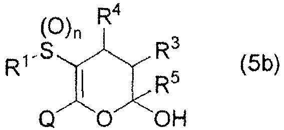 wherein the symbols have the same meanings as defined above, with ammonia or an ammonium salt under the presence of an alcohol represented by
R²OH
wherein the symbol has the same meaning as defined above, to obtain the compound represented by formula (2).

7. The method according to claim 5, which comprises a step of reacting a compound represented by formula (1): 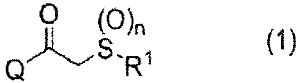 wherein the symbols have the same meaning as defined above, with ammonia or an ammonium salt to obtain the compound represented by formula (6).

8. The method according to claim 6, which comprises a step of reacting a compound represented by formula (1): 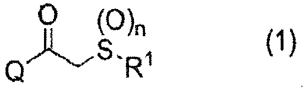 wherein the symbols have the same meanings as defined above, with a compound represented by formula (3): 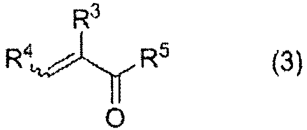 wherein the symbols have the same meaning as defined above, to obtain the compound represented by formula (5a) or the compound represented by formula (5b).
